# EUROPEAN PATENT APPLICATION

(11) **EP 4 772 144 A1**
(43) Date of publication of application: **08.07.2026**
(21) Application number: 25737532.9
(22) Date of filing: 29.04.2025
(51) Int. Cl.: A61F 2/24

(54) **SINGLE-LEAFLET MECHANICAL VALVE**

(30) Priority: 19.03.2025 CN 202510322979
(71) Applicant: Trio Metal (GZ) Co., Ltd, Guangzhou, Guangdong 510000 (CN); The First Affiliated Hospital of Xiamen University, Xiamen, Fujian 361000 (CN)
(72) Inventor: SHAN, Zhonggui, Xiamen, Fujian 361000 (CN); ZHOU, Xiaobiao, Xiamen, Fujian 361000 (CN); WANG, Jiamao, Xiamen, Fujian 361000 (CN); ZENG, Bin, Guangzhou, Guangdong 510000 (CN); ZU, Xiaoming, Guangzhou, Guangdong 510000 (CN)
(74) Representative: Plavsa, Olga
(86) International application number: PCT/CN2025/091925
(87) International publication number: WO 2026/026066

(57) **Abstract**

The present disclosure discloses a monoleaflet mechanical valve, which includes a mechanical valvular annulus, a limiting base, a movable valve leaflet and a guide rod; the mechanical valvular annulus is provided with a through-via in the middle; the limiting base is mounted on the bottom of the mechanical valvular annulus, and an open movement area is defined between the mechanical valvular annulus and the limiting base; both the movable valve leaflet and the guide rod are located in the movement area, the movable valve leaflet can reciprocate up and down along the trajectoy direction of the guide rod, such that the movable valve leaflet can movably open or close an entire passage opening of the through-via, so as to realize the relative increasing of the orifice area and a small transvalvular pressure gradient. According to the present disclosure, by means of the cooperation between the movable valve leaflet and the guide rod, thrombosis is effectively prevented, and the monoleaflet mechanical valve cannot be easily damaged. Even if thrombi are formed, the monoleaflet mechanical valve cannot easily get stuck, avoiding the risk of valve sticking or dysfunction, and the anticoagulant therapy can be reduced or is not needed. The mechanical valve provides a larger orifice area, significantly improving the performance of the valve, and therefore, the mechanical valve is suitable for more clinical patients, particularly patients with a small valvular annulus or underweight.

## Description

### TECHNICAL FIELD

The present disclosure relates to the technical field of mechanical valves, and in particular, to a monoleaflet mechanical valve.

### BACKGROUND

In patients with heart diseases, especially the pathological changes of the mitral valve, the aortic valve, the tricuspid valve and the pulmonary valve are often accompanied by congenital malformations of the valves or problems such as acquired stenosis and insufficiency, and in turn cause cardiac insufficiency, leading to congestions of systemic circulation or pulmonary circulation. Clinically, such patients show symptoms after exercise, such as asthma, palpitation, fatigue, abdominal distension and lower extremity edema. If not treated , the diseases may deteriorate rapidly and even develop into heart failure, and as a result, the patients can only choose artificial heart implantation or wait for heart transplantation. At present, the main treatment methods for valve diseases include valve repair and valve replacement surgeries.

In valve replacement, artificial valves clinically widely used are divided into mechanical valves and biological valves. For patients without atrial fibrillation, the anticoagulant therapy usually lasts for half a year after biological valve replacement. However, the service life of biological valves is limited, generally 3 to 15 years, and with the increase of service time, there is a risk of failure and damage, so the biological valves are not suitable for young patients in particular. Mechanical valves have become a common choice because of their long-term durability, but lifelong anticoagulant therapy is required. No matter whether the mechanical valves are monoleaflet, bileaflet or trileaflet mechanical valves, there are risks of visceral hemorrhages, such as cerebral hemorrhage and alimentary tract hemorrhage, pannus overgrowth, valve orifice area loss and even dysfunction during anticoagulant therapy, which require reoperation. Moreover, insufficient anticoagulation may lead to thrombosis, resulting in valve dysfunction or embolism, which further increases the risk of complications such as valve sticking and cerebral infarction. For some patients with a small valvular annulus or underweight, conventional mechanical valves and biological valves cannot meet the requirements of implantation because of the small size of the valvular annulus. For this type of patients, it is usually necessary to enlarge the valvular annulus or choose an oversized valve for implantation, which not only increases the difficulty of operation, but also leads to a significant increase in transvalvular pressure gradient, affecting the cardiac function of the patients and possibly shortening the service life of the valve. Particularly in patients with a small valvular annulus, oversized valves increase the risk of valve dysfunction, which in turn affects the therapeutic effect.

Therefore, there are still many problems when conventional mechanical valves and biological valves are applied to patients with a small valvular annulus or underweight, and there is an urgent need for a novel valve that can solve these limitations.

### SUMMARY

The objective of the present disclosure is to provide a monoleaflet mechanical valve which is intended to solve the aforementioned problems, and in particular, to provide a mechanical valve which can be used for a long time and avoid valve sticking or dysfunction, does not need anticoagulant therapy or only needs minor anticoagulant therapy, and has a larger valve orifice area (the orifice area of the valve of the same model is significantly larger than those of conventional mechanical valves and biological valves).

In order to achieve the aforementioned objective, the technical solution adopted by the present disclosure is as follows:

The present application provides a monoleaflet mechanical valve, including:
a mechanical valvular annulus, provided with a through-via in the middle;
a limiting base, mounted at the bottom of the mechanical valvular annulus, where an open movement area is defined between the mechanical valvular annulus and the limiting base, and the top of the movement area is communicated with the through-via to form a blood passage;
a movable valve leaflet and a guide rod, both located in the movement area, where the movable valve leaflet has a circular periphery and a top convex surface, such that blood flow resistance and thrombus adhesion can be reduced, and the movable valve leaflet is mounted on the guide rod and can reciprocate up and down along the trajectoy direction of the guide rod, such that the movable valve leaflet can movably open or close an entire passage opening of the through-via, so as to realize the relative increasing of the orifice area and a small transvalvular pressure gradient.

In a possible embodiment, a middle portion of the movable valve leaflet is provided with a sliding hole which is matched with the guide rod for sliding, and the guide rod is inserted into the sliding hole, such that the movable valve leaflet can slide up and down in a reciprocating manner along the trajectoy direction of the guide rod, which can reduce thrombosis and valve sticking dysfunction.

In a possible embodiment, the inner side of the mechanical valvular annulus is arc-shaped.

In a possible embodiment, the limiting base is of an annular hollow structure, and a first supporting component for supporting the guide rod is connected within an inner diameter of the limiting base, and a bottom end of the guide rod is mounted on the first supporting component.

In a possible embodiment, the first supporting component includes a first connecting part, an arched section and a second connecting part which are connected in sequence;
a first end of the first connecting part is connected to one side of an open end of the arched section, and a first end of the second connecting part is connected to the other side of the open end of the arched section;
the second end of the first connecting part and the second end of the second connecting part are connected to the limiting base respectively, and the bottom end of the guide rod is mounted on a middle portion of the arched section.

In a possible embodiment, the movable valve leaflet is internally provided with an arched cavity, and an outer curving radian of the arched section is equal to or smaller than a curving radian of the arched cavity, so as to support the movable valve leaflet sliding down along the guide rod.

In a possible embodiment, the first supporting component is a cross bar, a first end of the first supporting component is connected to the limiting base, and a second end of the first supporting component is located on a middle portion of the limiting base and connected to the bottom end of the guide rod.

In a possible embodiment, the top of the guide rod is higher than the mechanical valvular annulus, such that the movable valve leaflet has a movement allowance section along the moving trajectoy direction of the guide rod.

In a possible embodiment, a second supporting component for mounting the guide rod is connected within an inner diameter of the mechanical valvular annulus, and the top of the guide rod is mounted on a middle portion of the second supporting component.

In a possible embodiment, the second supporting component is of an arc-shaped or elliptic structure.

In a possible embodiment, at least one supporting leg is connected between the bottom of the mechanical valvular annulus and the top of the limiting base, to form the open movement area.

In a possible embodiment, the outer diameter of the movable valve leaflet is larger than the inner diameter of the through-via and smaller than the inner diameter of the movement area defined by the supporting leg.

In a possible embodiment, the top convex surface of the movable valve leaflet may extend to the outside of the mechanical valvular annulus.

In a possible embodiment, an annular suture ring is coaxially arranged on an outer side of the mechanical valvular annulus.

To sum up, due to the adoption of the technical solution, the present disclosure has the following beneficial effects:

In the present disclosure, by means of the cooperation between the movable valve leaflet and the guide rod, the movable valve leaflet can reciprocate up and down, and therefore, compared with conventional mechanical valves, the monoleaflet mechanical valve can effectively prevent thrombosis, cannot be easily damaged, and can be used for a long time; and even if thrombi are formed, the monoleaflet mechanical valve cannot easily get stuck, thus avoiding valve sticking or dysfunction. Because thrombi cannot be easily formed, a patient does not need anticoagulant therapy or only needs minor anticoagulant therapy during use. Because no mechanical valve leaflet or biological valve obstructs the through-via, compared with conventional valves of the same model, the monoleaflet mechanical valve has an orifice area which is significantly larger than those of conventional mechanical valves and biological valves, providing a larger orifice area and a small transvalvular pressure gradient, significantly improving the performance of the valve, and therefore, the monoleaflet mechanical valve is suitable for more clinical patients, particularly patients with a small valvular annulus or underweight.

### BRIEF DESCRIPTION OF THE DRAWINGS

FIG. 1 is a schematic three-dimensional diagram of the present disclosure;
FIG. 2 is a sectional view of a movable valve leaflet and a through-via in a closed state according to the present disclosure;
FIG. 3 is a three-dimensional diagram of the movable valve leaflet and the through-via in a opened state according to the present disclosure;
FIG. 4 is a sectional view of the movable valve leaflet and the through-via in the opened state according to the present disclosure;
FIG. 5 is a schematic three-dimensional diagram of the movable valve leaflet according to the present disclosure;
FIG. 6 is a sectional view of the movable valve leaflet according to the present disclosure;
FIG. 7 is a three-dimensional diagram of another embodiment of a mechanical valve according to the present disclosure; and
FIG. 8 is a sectional view of another embodiment of the mechanical valve according to the present disclosure.

### Reference numerals:

1. mechanical valvular annulus; 101. through-via; 2. limiting base; 201. movement area; 3. movable valve leaflet; 301. sliding hole; 302. convex surface; 303. arched cavity; 4. guide rod; 400. movement allowance section; 5. first supporting component; 501. first connecting part; 502. arched section; 503. second connecting part; 6. second supporting component; 7. supporting leg; 8. annular suture ring.

### DETAILED DESCRIPTION

In order to make the objective, technical solutions and advantages of the present application clearer, implementations of the present application will be further described in detail below with reference to the accompanying drawings.

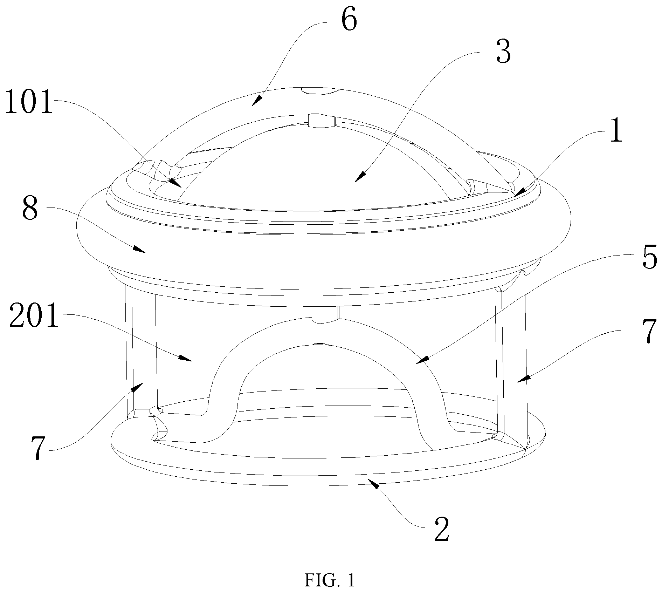

First, the overall structure of a monoleaflet mechanical valve will be illustrated with reference to FIG. 1. FIG. 1 is a schematic three-dimensional diagram of the present disclosure. The application of the monoleaflet mechanical valve in clinical conditions will be illustrated hereinafter.

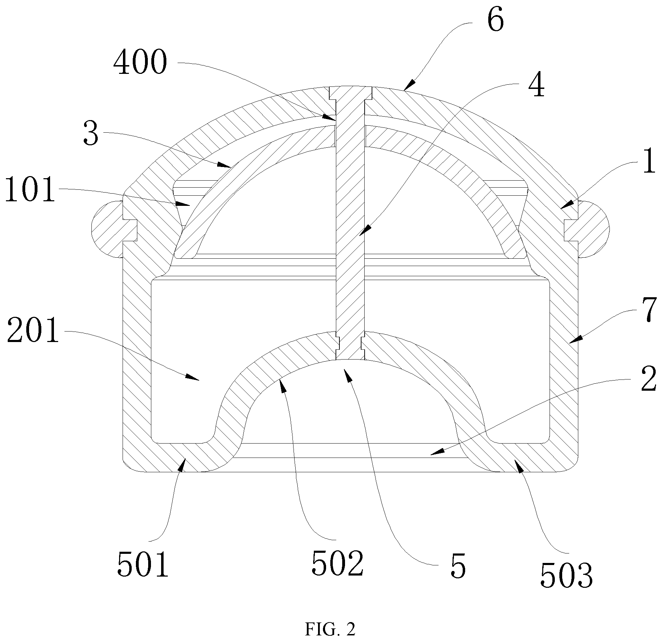

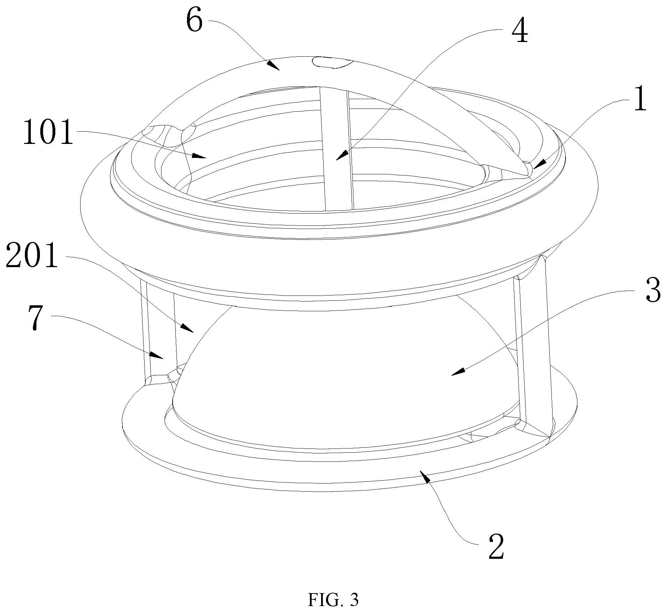

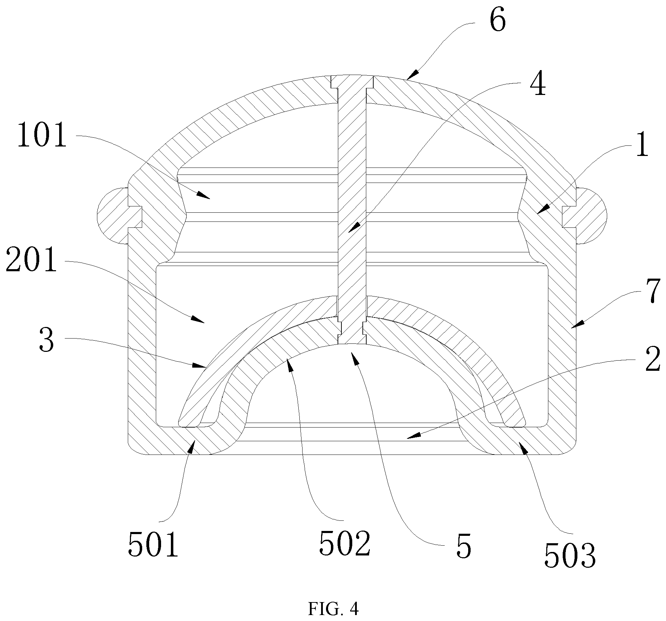

Referring to FIGs. 2 to 4, FIG. 2 is a sectional view of a movable valve leaflet 3 and a through-via 101 in a closed state according to the present disclosure. FIG. 3 is a three-dimensional diagram of the movable valve leaflet 3 and the through-via 101 in a opened state according to the present disclosure. FIG. 4 is a sectional view of the movable valve leaflet 3 and the through-via 101 in the opened state according to the present disclosure.

The monoleaflet mechanical valve includes a mechanical valvular annulus 1, a limiting base 2, a movable valve leaflet 3 and a guide rod 4, where:

Referring to FIG. 1, the mechanical valvular annulus 1 is generally circular or elliptical, and is made of metal such as titanium alloy. When the mechanical valve is implanted in the human body, for example, when the mechanical valve is implanted for replacement into the tricuspid valve or mitral valve of the heart, the mechanical valvular annulus 1 is fixed at the autologous valvular annulus. A middle portion of the mechanical valvular annulus 1 is provided with a through-via 101 for blood circulation. Because the inner side of the mechanical valvular annulus 1 is shaped like an arc with a smooth edge, blood flow resistance is low, the transvalvular pressure gradient is small, and thrombi cannot easily adhere.

The limiting base 2 is located in a ventricle. The limiting base 2 is of an annular hollow structure, and an open movement area 201 is arranged between the mechanical valvular annulus 1 and the limiting base 2, and the limiting base 2 is mounted at the bottom of the mechanical valvular annulus 1. As shown in FIG. 3, the top of the movement area 201 is communicated with the through-via 101 to form a blood passage.

Referring to FIGs. 1 to 4, the movable valve leaflet 3 is fitted on the guide rod 4. Both the movable valve leaflet 3 and the guide rod 4 are located in the movement area 201. The movable valve leaflet 3 is mounted on the guide rod 4 and can reciprocate up and down along the trajectoy direction of the guide rod 4, such that the movable valve leaflet 3 can movably open an entire passage opening of the through-via 101 (see FIGs. 3 and 4), or the movable valve leaflet 3 can movably close the entire passage opening of the through-via 101 (see FIGs. 1 and 2), so as to realize the relative increasing of the orifice area (the orifice area of the valve of the same model is significantly larger than those of conventional mechanical valves and biological valves) and a small transvalvular pressure gradient, significantly improving the performance of the valve, and therefore, the monoleaflet mechanical valve is suitable for more clinical patients, particularly patients with a small valvular annulus or underweight.

In the present embodiment, after the mechanical valve is implanted into the human body, when the ventricle contracts, the movable valve leaflet 3 moves to the mechanical valvular annulus 1 along the guide rod 4 to close the through-via 101 (referring to FIGs. 1 and 2), such that the mechanical valvular annulus 1 is closed, that is, the blood passage is obstructed, preventing the blood flow from reaching an atrium via the mechanical valvular annulus 1;

When the atrium contracts, the movable valve leaflet 3 moves away from the mechanical valve annulus 1 along the guide rod 4, such that there is no obstruction at the through-via 101 (see FIGs. 3 and 4), making the blood channel unobstructed, and therefore, the blood can flow from the atrium to the ventricle in one direction, realizing a unidirectional circulation function of the blood passage.

In the present disclosure, by means of the cooperation between the movable valve leaflet 3 and the guide rod 4, the movable valve leaflet 3 can reciprocate up and down. Compared with conventional mechanical valves, the monoleaflet mechanical valve has no slot, shaft and other parts in structure, effectively preventing thrombosis and prolonging the service life of the mechanical valve, and therefore, the monoleaflet mechanical valve cannot be easily damaged and can be used for a long time. Even if thrombi are formed, the monoleaflet mechanical valve cannot easily get stuck, avoiding valve sticking or dysfunction. Moreover, because thrombi cannot be easily formed, a patient does not need anticoagulant therapy or only needs minor anticoagulant therapy during use.

In addition, for some patients with a small valvular annulus or underweight, since the valvular annulus is small, the current mechanical valves and biological valves cannot be normally implanted when applied, but often need to be implanted by tilting the valve, enlarging the valvular annulus or adopting other methods, and the transvalvular pressure gradient becomes high, affecting the cardiac function of the patients and the service life of the valves.

In view of the aforementioned problems, referring to FIGs. 3 and 4, when the aforementioned movable valve leaflet 3 moves down along the guide rod 4 to open the through-via 101, no mechanical valve leaflet or biological valve obstructs the through-via 101 (i.e., the valve orifice). Compared with conventional valves of the same model, the orifice area of the mechanical valve in the present disclosure is significantly larger than those of conventional mechanical valves and biological valves, providing a larger orifice area and a smaller transvalvular pressure gradient. Therefore, for patients with a small valvular annulus, a model which is smaller than currently applied valves can be chosen for implantation, and a smaller transvalvular pressure gradient can also be achieved without tilting the valve, enlarging the valvular annulus or adopting other methods for implantation.

Of course, the mechanical valve can also be implanted for replacement into the aortic valve or the pulmonary valve, such that the limiting base 2 is located in the aorta or the pulmonary artery, playing a role of unidirectional circulation along the blood flow direction.

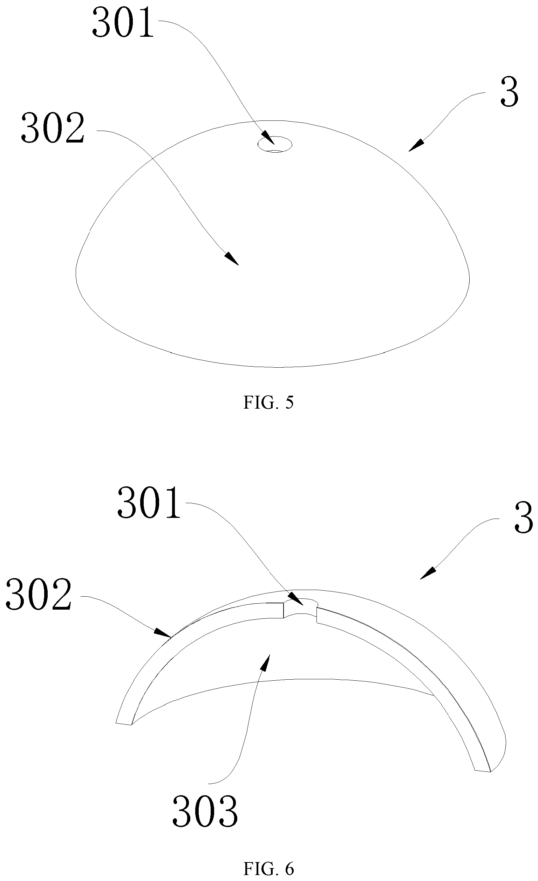

In some embodiments, as shown in FIG. 5, FIG. 5 is a schematic three-dimensional diagram of the movable valve leaflet 3 according to the present disclosure. With regard to the specific structure of the movable valve leaflet 3 and the principle of cooperation between the movable valve leaflet 3 and the guide rod 4, specifically,
the movable valve leaflet 3 has a circular periphery and a top convex surface 302, such that blood flow resistance and thrombus adhesion can be reduce;
the middle portion of the movable valve leaflet 3 is provided with a sliding hole 301, which is matched with the guide rod 4 for sliding, and the guide rod 4 is inserted into the sliding hole 301, such that the movable valve leaflet 3 can slide up and down in a reciprocating manner along the trajectoy direction of the guide rod 4.

In the present embodiment, as shown in FIG. 5, the movable valve leaflet 3 is designed to have a circular periphery and a top convex surface 302. Thanks to such a design, the flow resistance of the blood in contact with the movable valve leaflet 3 is low, the transvalvular pressure gradient is small, and thrombi cannot easily adhere as well. Moreover, since the movable valve leaflet 3 can vertically slide along the central axis of the guide rod 4, the monoleaflet mechanical valve cannot be damaged, and has a long service life and no risk of thrombosis, valve sticking, dysfunction, etc. Therefore, the frequency of anticoagulant therapy can be reduced.

In addition, due to the mechanical characteristics of mechanical valves, some patients can hear the closing sound "click" of the mechanical valves when the mechanical valves installed in the hearts is opened and closed, which will bring a bad feeling to some people. In view of the problem of the sound "click", as shown in FIGs. 1 and 2, the dome-shaped movable valve leaflet 3 is a whole. When the movable valve leaflet 3 is changed from an opened state to a closed state, the entire dome opening of the movable valve leaflet 3 will be fastened with the mechanical valvular annulus 1, thus preventing a certain point or certain area of the movable valve leaflet 3 from strongly impacting the mechanical valvular annulus to generate sound. Since the performance of smooth closing is improved, the noise of the operation of the movable valve leaflet 3 is greatly reduced.

In some embodiments, as shown in FIG. 1, FIG. 1 is a schematic three-dimensional diagram of the present disclosure. With regard to the specific mounting structure of the guide rod 4, A first supporting component 5 for supporting the guide rod 4 is connected within an inner diameter of the limiting base 2, and both sides of the first supporting component 5 are provided with open areas for blood circulation, such that the limiting base 2 forms a hollow structure.

A bottom end of the guide rod 4 is mounted on the first supporting component 5, which plays a role of supporting and mounting the guide rod 4.

As shown in FIG. 2, FIG. 2 is a sectional view of a movable valve leaflet 3 and a through-via 101 in a closed state according to the present disclosure. With regard to the specific structure and operating principle of the first supporting component 5, the first supporting component 5 includes a first connecting part 501, an arched section 502 and a second connecting part 503 which are connected in sequence.

A first end of the first connecting part 501 is connected to one side of an open end of the arched section 502, and a first end of the second connecting part 503 is connected to the other side of the open end of the arched section 502.

The second end of the first connecting part 501 and the second end of the second connecting part 503 are connected to the limiting base 2 respectively, such that the arched section 502 is higher than the limiting base 2, and the bottom end of the guide rod 4 is mounted on the middle portion of the arched section 502.

As shown in FIG. 6, FIG. 6 is a sectional view of the movable valve leaflet 3 according to the present disclosure. In the present embodiment, the movable valve leaflet 3 is internally provided with an arched cavity 303, and an outer curving radian of the arched section 502 is equal to or smaller than a curving radian of the arched cavity 303. In the present embodiment, when the movable valve leaflet 3 moves down along the trajectoy direction of the guide rod 4, the arched cavity 303 arranged in the movable valve leaflet 3 is used to support the movable valve leaflet 3 sliding down along the guide rod 4, such that valve sticking can be further avoided.

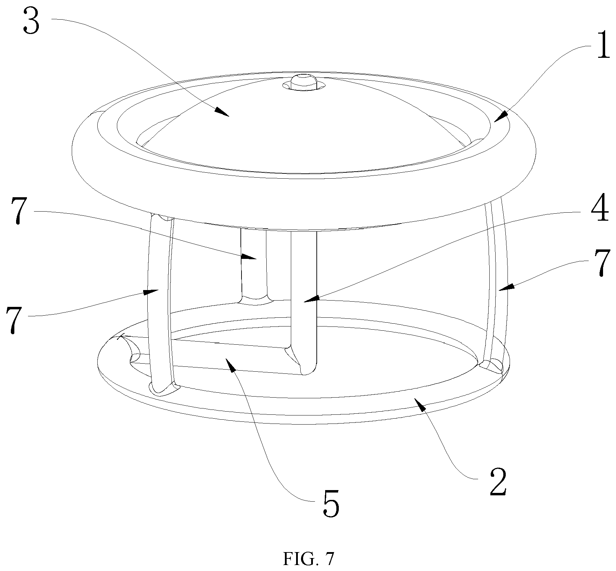

In some embodiments, as shown in FIG. 7, FIG. 7 is a three-dimensional diagram of another embodiment of a mechanical valve according to the present disclosure. The difference between the structure of the mechanical valve and that in the aforementioned embodiment is that the first supporting component 5 is a cross bar, a first end of the first supporting component 5 is connected to the limiting base 2, a second end of the first supporting component 5 is located on the middle portion of the limiting base 2 and connected to the bottom end of the guide rod 4, such that the first supporting component 5 is arranged perpendicular to the guide rod 4, and the first supporting component 5 and the guide rod 4 play a role as a whole for supporting and guiding the movable valve leaflet 3.

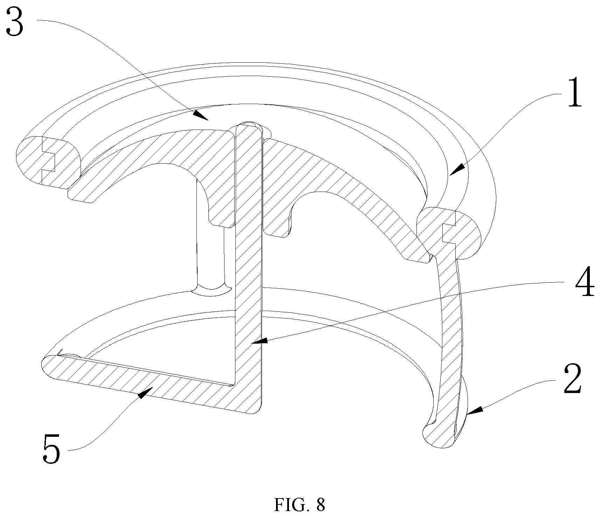

In addition, as shown in FIG. 8, FIG. 8 is a sectional view of another embodiment of the mechanical valve according to the present disclosure. In the present embodiment, the bottom of the movable valve leaflet 3 is of a solid surface structure, and the bottom of the movable valve leaflet 3 may also be provided with an arched cavity 303. Thanks to the arrangement of the arched cavity 303, the blood flow can smoothly push the movable valve leaflet 3 to block the through-via 101 when the ventricle contracts, and the consumption of material can be reduced, reducing the cost and the weight of the movable valve leaflet 3.

In some embodiments, as shown in FIG. 1, FIG. 1 is a schematic three-dimensional diagram of the present disclosure. With regard to the connection structure between the limiting base 2 and the mechanical valvular annulus 1, specifically, at least one supporting leg 7 is connected between the bottom of the mechanical valvular annulus 1 and the top of the limiting base 2. As shown in FIG. 1, there are two supporting legs 7, which are symmetrically arranged between the mechanical valvular annulus 1 and the limiting base 2. Alternatively, as shown in FIG. 7, there are three supporting legs 7, which are arranged in the form of an annular matrix between the mechanical valvular annulus 1 and the limiting base 2. The space between the mechanical valvular annulus 1 and the limiting base 2 constitutes the movement area 201.

In addition, as shown in FIG. 1 or 7, The outer diameter of the movable valve leaflet 3 is larger than the inner diameter of the through-via 101, and the outer diameter of the movable valve leaflet 3 is smaller than the inner diameter of the movement area 201 defined by the supporting legs 7. Thus, the movable valve leaflet 3 can freely move up and down in the movement area 201.

In some embodiments, as shown in FIG. 1, FIG. 1 is a schematic three-dimensional diagram of the present disclosure. A second supporting component 6 for mounting the guide rod 4 is connected within an inner diameter of the mechanical valvular annulus 1, and the second supporting component 6 is of an arc-shaped or elliptic structure.

The top of the guide rod 4 is mounted on the middle portion of the second supporting component 6. The top of the guide rod 4 is higher than the mechanical valvular annulus 1, such that the movable valve leaflet 3 has a movement allowance section 400 along the moving trajectoy direction of the guide rod 4. When the movable valve leaflet 3 moves up along the guide rod 4 to close the through-via 101, the top convex surface 302 of the movable valve leaflet 3 can extend to the outside of the mechanical valvular annulus 1.

In the present embodiment, the mechanical valve is of a hollow structure, the guide rod 4 is fixed between the first supporting component 5 and the second supporting component 6, and part of the mechanical valve protrudes from the atrium. Therefore, the total volume is small, and the blood volume in the atria, ventricles or blood vessels is not affected.

In some embodiments, as shown in FIG. 1, FIG. 1 is a schematic three-dimensional diagram of the present disclosure. In order to facilitate the fixed connection between the mechanical valvular annulus 1 and the autologous valvular annulus, an annular suture ring 8 is coaxially arranged on an outer side of the mechanical valvular annulus 1. The mechanical valvular annulus 1 and the autologous valve can be conveniently sutured and fixed by the annular suture ring 8. The structure of that annular suture ring 8 is a conventional structure.

In the description of hte present disclosure, it should be noted that the term "comprise", "include" or any other variant thereof herein is intended to cover non-exclusive inclusion, such that a process, method, article or device including a series of elements includes not only those elements but also other elements not explicitly listed or elements inherent to such process, method, article or device. Without more restrictions, the elements defined by the sentence "include a ..." do not exclude the presence of other identical elements in the process, method, article or device including the elements.

In addition, in the description of the present disclosure, directions or positional relations indicated by terms, such as "upper", "lower", "front", "rear", "left", "right", "top", "bottom", "inner", "outer", etc., are directions or positional relations shown in the accompanying drawings, which are merely intended to conveniently describe the present disclosure and simplify description rather than indicate or imply that the indicated device or elements must have specific directions and be structured and operated according to the specific directions, and therefore should not be interpreted as limitations on the present disclosure.

In another aspect, it should be noted that unless clearly specified and defined otherwise, terms, such as "arrange", "mount", "interconnect" and "connect", should be broadly comprehended as "fixedly connect", "detachably connect" or "integrally connect"; "mechanically connect" or "electrically connect"; or "directly interconnect", "indirectly interconnect through an intermediate" or "the interiors of two elements communicate with each other". As used herein, the reference to a "component", "part" or "portion" should not be limited to a single structural component, part or element, but may include an assembly of components, parts or elements. For those of ordinary skill in the art, the specific meanings of the above terms in the present disclosure can be understood according to specific conditions.

## Claims

1. A monoleaflet mechanical valve, comprising:
a mechanical valvular annulus, provided with a through-via in the middle;
a limiting base, mounted at the bottom of the mechanical valvular annulus, wherein an open movement area is defined between the mechanical valvular annulus and the limiting base, and the top of the movement area is communicated with the through-via to form a blood passage;
a movable valve leaflet and a guide rod, both located in the movement area, wherein the movable valve leaflet has a circular periphery and a top convex surface, and the movable valve leaflet is mounted on the guide rod and can reciprocate up and down along a trajectoy direction of the guide rod, such that the movable valve leaflet can movably open or close an entire passage opening of the through-via.

2. The monoleaflet mechanical valve according to claim 1, wherein a middle portion of the movable valve leaflet is provided with a sliding hole which is matched with the guide rod for sliding, and the guide rod is inserted into the sliding hole, such that the movable valve leaflet can slide up and down in a reciprocating manner along the trajectoy direction of the guide rod.

3. The monoleaflet mechanical valve according to claim 1 or 2, wherein the limiting base is of an annular hollow structure, and a first supporting component for supporting the guide rod is connected within an inner diameter of the limiting base, and a bottom end of the guide rod is mounted on the first supporting component.

4. The monoleaflet mechanical valve according to claim 3, wherein the first supporting component comprises a first connecting part, an arched section and a second connecting part which are connected in sequence;
a first end of the first connecting part is connected to one side of an open end of the arched section, and a first end of the second connecting part is connected to the other side of the open end of the arched section;
the second end of the first connecting part and the second end of the second connecting part are connected to the limiting base respectively, and the bottom end of the guide rod is mounted on a middle portion of the arched section.

5. The monoleaflet mechanical valve according to claim 4, wherein the movable valve leaflet is internally provided with an arched cavity, and an outer curving radian of the arched section is equal to or smaller than a curving radian of the arched cavity, so as to support the movable valve leaflet sliding down along the guide rod.

6. The monoleaflet mechanical valve according to claim 3, wherein the first supporting component is a cross bar, a first end of the first supporting component is connected to the limiting base, and a second end of the first supporting component is located in the middle of the limiting base and connected to the bottom end of the guide rod.

7. The monoleaflet mechanical valve according to claim 4, wherein a second supporting component for mounting the guide rod is connected within an inner diameter of the mechanical valvular annulus, and the top of the guide rod is mounted on a middle portion of the second supporting component.

8. The monoleaflet mechanical valve according to claim 7, wherein the top of the guide rod is higher than the mechanical valvular annulus, such that the movable valve leaflet has a movement allowance section along the moving trajectoy direction of the guide rod.

9. The monoleaflet mechanical valve according to claim 7, wherein at least one supporting leg is connected between the bottom of the mechanical valvular annulus and the top of the limiting base, to form the open movement area; and
the outer diameter of the movable valve leaflet is larger than the inner diameter of the through-via and smaller than the inner diameter of the movement area defined by the supporting leg.

10. The monoleaflet mechanical valve according to claim 1, wherein the top convex surface of the movable valve leaflet can extend to the outside of the mechanical valvular annulus.

11. The monoleaflet mechanical valve according to claim 1, wherein an annular suture ring is coaxially arranged on an outer side of the mechanical valvular annulus.
